# EUROPEAN PATENT APPLICATION

(11) **EP 4 628 149 A1**
(43) Date of publication of application: **08.10.2025**
(21) Application number: 24168869.6
(22) Date of filing: 05.04.2024
(51) Int. Cl.: A61N 1/05, A61N 1/368, A61N 1/372

(54) **SYSTEM FOR PROVIDING STIMULATION TO A PATIENT**

(71) Applicant: BIOTRONIK SE & Co. KG, 12359 Berlin (DE)
(72) Inventor: DÖRR, Thomas, 12437 Berlin (DE)
(74) Representative: Biotronik Corporate Services SE

(57) **Abstract**

The present disclosure relates to a system (100) for providing stimulation to a patient, in particular stimulation to a left bundle branch area of the heart of the patient, the system comprising: a stimulation pulse generator (10); at least one electrode (30) having a distal end for being engaged with the patient and a proximal end for being engaged with the generator, the electrode being configured to be implanted at least partially in the septum of the heart of the patient, and to provide stimulation to the patient, wherein the distal end comprises a fixing portion configured to facilitate implanting in the septum; a sheath (50) having a three-dimensional opening for guiding the at least one electrode for implanting of the electrode; wherein the generator, the electrode and the sheath are adapted for use in combination with each other.

## Description

### Technical field

The present invention relates to a system, method, and computer program for providing stimulation to a patient. More specifically, the invention pertains to a system and method for providing targeted electrical stimulation to the left bundle branch area of the heart to enhance cardiac function and treat various cardiac conditions.

### Technical background

Cardiac rhythm management devices, such as pacemakers and implantable cardioverter-defibrillators (ICDs), have been developed to provide electrical stimulation to the heart in order to treat arrhythmias and to improve cardiac function. Traditional pacing systems typically stimulate the heart at the right atrium and/or right ventricle to induce contraction. However, this can result in non-physiological activation patterns, potentially leading to or exacerbating heart failure due to dyssynchronous ventricular contraction.

Cardiac resynchronization therapy (CRT) has emerged as a valuable means for heart failure patients with ventricular dyssynchrony, typically involving the stimulation of both the left and right ventricles to resynchronize cardiac contractions. Traditionally, CRT is accomplished using epicardial leads placed on the surface of the heart or by transvenous leads positioned within the coronary sinus to indirectly stimulate the left ventricle.

Recent advances have focused on more physiological pacing techniques, such as direct left bundle branch area pacing, to mimic the heart's natural conduction pathway. Pacing the left bundle branch area shows promise in providing a more synchronized activation of the ventricles and improving cardiac function, yet existing systems and methods still face various challenges. In particular the prior art lacks medically approved systems that cover all necessary components in order to smoothly perform implantation and stimulation of the left bundle branch area.

The present invention seeks to address these issues by introducing improvements over the known systems and methods.

### Summary of the invention

The above-mentioned objects and other objects, which become apparent from the following description, are solved by the subject-matter of the independent claims. Preferred embodiments are subject of the dependent claims.

A first aspect of the disclosure is related to a system for providing stimulation to a patient, in particular stimulation to a left bundle branch area of the heart of the patient, the system comprising: a stimulation pulse generator; at least one electrode having a distal end for being engaged with the patient and a proximal end for being engaged with the generator, the electrode being configured to be implanted at least partially in the septum of the heart of the patient, and to provide stimulation to the patient, wherein the distal end comprises a fixing portion configured to facilitate implanting in the septum; a sheath having a three-dimensional opening for guiding the at least one electrode for implanting of the electrode; wherein the generator, the electrode and the sheath are adapted for use in combination with each other.

The system has the advantage to provide a combined set of components, i.e., a generator, an electrode, and a sheath that can be easily used for implanting and stimulating. Further, as these components are adapted for use in combination with each other, they allow to be medically approved. This therefore provides advantages over known individual components, which necessitate off-label usage. The system has the further advantage to enhance cardiac function and treat various cardiac conditions. The system may be a conduction-system-pacing-system.

In a further embodiment of the system, the generator may comprise at least one predefined parameter configured to indicate a position of the electrode, preferably of the distal end of the electrode when implanted in the septum of the heart.

In a further embodiment of the system, the generator may comprise stimulation and/or diagnostic functions that are configured to be dependent upon a location of the distal end of the electrode.

In a further embodiment of the system, the fixing portion may comprise a helical structure having a length of 0.1 to 5 mm, preferably 0.5 to 4 mm, preferably 1 to 3 mm, most preferably 1.5 to 1.8 mm, the helical structure configured to allow the distal end of the electrode to be screwed in the septum, wherein the helical structure is preferably a piece separate from the electrode such that the helical structure can be at least partially removably attached to the electrode.

In a further embodiment of the system, the fixing portion may comprise a first pole and the electrode comprises a second pole in proximity of the distal end, preferably between 1 to 10 mm, preferably 2 to 9 mm, preferably 3 to 8 mm, preferably 4 to 7 mm, preferably 5 to 6 mm spaced apart from the distal end, the first and the second pole configured to provide stimulation to the patient, wherein the electrode is preferably a bipolar electrode, wherein the second pole has preferably the shape of a ring, preferably having a length along the longitudinal direction of the electrode of at least 0.5 mm, preferably at least 1 mm, preferably at least 1.5 mm, preferably at least 2 mm and/or of at most 5 mm, preferably at most 4 mm, preferably at most 3 mm, preferably at most 2 mm.

In a further embodiment of the system, the sheath may have an inner surface facing the opening, wherein the inner surface is configured so as to facilitate guiding the electrode.

In a further embodiment of the system, the stimulation may be configured to provide left bundle branch area pacing, wherein the system is preferably a conduction system pacing system.

In a further embodiment of the system, the generator may be one or more of an implantable pulse generator, IPG, an implantable cardioverter defibrillators, ICD, a generator for cardiac resynchronization therapy with a defibrillator, CRT-D, a generator for cardiac resynchronization therapy with a pacemaker, CRT-P.

In a further embodiment of the system, the generator may comprise a recess for being engaged with the proximal end of the electrode, the recess comprising a first and a second pole for providing the stimulation to the electrode when the electrode is engaged with the recess, wherein the generator preferably comprises a second recess for being engaged with a further electrode, the second recess comprising a terminal, preferably an IS-1 terminal, wherein the further electrode is preferably an electrode configured to be implanted to provide stimulation to a left bundle branch area of the heart of the patient or not to provide stimulation to a left bundle branch area of the heart of the patient.

In a further embodiment of the system, the generator may comprise a programming interface comprising a switch configured to indicate whether or not the electrode is implanted in the left bundle branch area of the heart of the patient, wherein the switch is configured to be activated when the electrode is implanted in the left bundle branch area of the heart of the patient.

In a further embodiment of the system, when the switch is activated, the information that the electrode is implanted in the left bundle branch area of the heart of the patient is configured to be indicated to a human, such as the patient, wherein preferably a brady parameter screen and/or a follow-up screen is/are configured to display this information, wherein this information is preferably configured to be made available on programmer printouts, wherein this information is preferably configured to be uploaded to remote monitoring system, HMSC.

In a further embodiment of the system, when the switch is activated, a human such as the patient is allowed to enter left bundle branch, LBB, myocardial-only and/or Bundle branch block, BBB, correction capture threshold(s) separately or in combination for a substantially manual LV-threshold-test.

In a further embodiment of the system, providing stimulation to the patient is dependent on the information.

In a further embodiment of the system, the opening of the sheath comprises a stylet and/or wherein the sheath comprises a stylet, wherein the sheath is preferably a catheter.

In a further embodiment of the system, the sheath comprises a first deformation in a first plane and a second deformation in a second plane, the second plane being different from the first plane, wherein the sheath is configured to be cut so as to facilitate removal of the sheath preferably after implanting the electrode, wherein preferably a tool can be used in order to cut the sheath.

A second aspect of the disclosure is related to a method for providing stimulation to a patient, in particular stimulation to a left bundle branch area of the heart of the patient, comprising: using the system as described in the present disclosure.

A third aspect of the disclosure is related to a method for providing stimulation to a patient, in particular stimulation to a left bundle branch area of the heart of the patient, comprising: providing a stimulation pulse generator; providing at least one electrode having a distal end for being engaged with the patient and a proximal end for being engaged with the generator, the electrode being configured to be implanted at least partially in the septum of the heart of the patient, and to provide stimulation to the patient, wherein the distal end comprises a fixing portion configured to facilitate implanting in the septum; providing a sheath having a three-dimensional opening for guiding the at least one electrode for implanting of the electrode; wherein the generator, the electrode and the sheath are adapted for use in combination with each other.

A fourth aspect of the disclosure is related to a method for implanting an electrode, preferably an electrode of the system as described in the present disclosure, comprising: placing a sheath at least partially in the septum of the heart of a user; guiding at least one electrode at least partially through the sheath, the electrode preferably having a distal end for being engaged with the patient and a proximal end for being engaged with a generator, the electrode being configured to be implanted at least partially in the septum of the heart of the patient, and to provide stimulation to the patient, implanting the electrode by at least partially fixing the electrode in the septum, preferably in the left bundle branch area of the heart of the user, optionally removing the sheath; optionally cutting the sheath.

A fifth aspect of the disclosure is related to a computer program comprising instructions which, when the program is executed by a computer, cause the computer to carry out any one of the methods described in here.

Any features and or advantages of the aspects as described in here can be combined with one another as understood by the skilled person.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium. Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor. Any means for controlling or the like as described herein may also be implemented in hardware, software, firmware, and/or combinations thereof, for example, by means of one or more general-purpose or special-purpose computers, and/or a general-purpose or special-purpose processors.

### Brief description of the figures

In the following, preferred embodiments are described, by way of example only. Reference is made to the following accompanying figures:
- Fig. 1: illustrates a system according to a first aspect;
- Fig. 2: illustrates a generator;
- Fig. 3: illustrates an electrode;
- Fig. 4: illustrates a sheath and an electrode; and
- Fig. 5: illustrates a method according to an embodiment.

### Detailed description of preferred embodiments

Subsequently, presently preferred embodiments will be outlined, primarily with reference to the above Figures. It is noted that further embodiments are certainly possible, and the below explanations are provided by way of example only, without limitation.

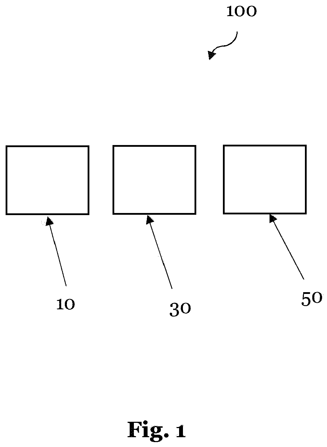

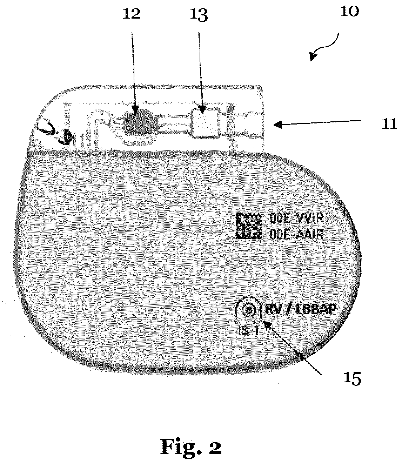

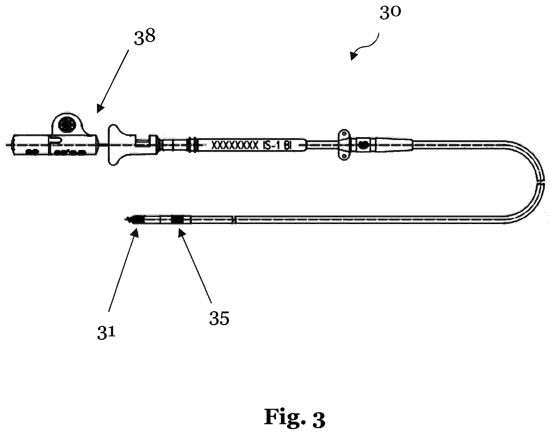

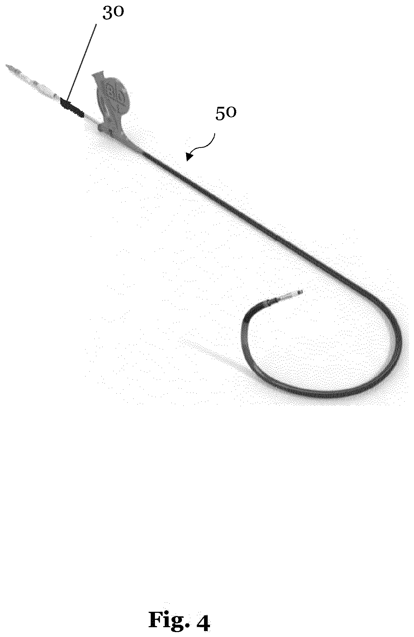

**Figs. 1 to 4** illustrates a system according to a first aspect. The system 100 is for providing stimulation to a patient, in particular stimulation to a left bundle branch area of the heart of the patient, the system 100 comprising: a stimulation pulse generator 10; at least one electrode 30 having a distal 31 end for being engaged with the patient and a proximal end 38 for being engaged with the generator 10, the electrode 30 being configured to be implanted at least partially in the septum of the heart of the patient, and to provide stimulation to the patient, wherein the distal end 31 comprises a fixing portion configured to facilitate implanting in the septum; a sheath 50 having a three-dimensional opening for guiding the at least one electrode 30 for implanting of the electrode 30; wherein the generator 10, the electrode 30 and the sheath 50 are adapted for use in combination with each other.

The sheath 50 may comprise an elongated shaft and/or it may have a helical curve. The sheath 50 may have a local radius of the helical curve increases along the sheath shaft from distal to proximal.

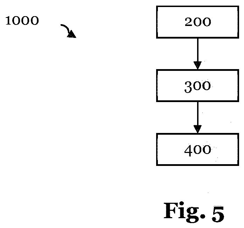

**Fig. 5** illustrates a method 1000 according to a fourth aspect of the disclosure, the method is for implanting an electrode, preferably an electrode of the system as described in the present disclosure. The method comprises: placing 200 a sheath at least partially in the septum of the heart of a user; guiding 300 at least one electrode at least partially through the sheath, the electrode preferably having a distal end for being engaged with the patient and a proximal end for being engaged with a generator, the electrode being configured to be implanted at least partially in the septum of the heart of the patient, and to provide stimulation to the patient, implanting 400 the electrode by at least partially fixing the electrode in the septum, preferably in the left bundle branch area of the heart of the user, optionally removing the sheath; optionally cutting the sheath.

It is noted that the above examples/embodiments may be combined with further aspects as described herein and details of the examples may also be omitted, as will be understood by the skilled person.

### Reference numerals

- 10: generator
- 11: recess
- 12: first pole of generator
- 13: second pole of generator
- 15: second recess, IS-1 terminal
- 30: electrode
- 31: distal end, helical structure
- 35: second pole
- 38: proximal end
- 50: sheath
- 100: system
- 1000: method
- 200: method step
- 300: method step
- 400: method step

## Claims

1. System (100) for providing stimulation to a patient, in particular stimulation to a left bundle branch area of the heart of the patient, the system comprising:
a stimulation pulse generator (10);
at least one electrode (30) having a distal end for being engaged with the patient and a proximal end for being engaged with the generator, the electrode being configured to be implanted at least partially in the septum of the heart of the patient, and to provide stimulation to the patient, wherein the distal end comprises a fixing portion configured to facilitate implanting in the septum;
a sheath (50) having a three-dimensional opening for guiding the at least one electrode for implanting of the electrode;
wherein the generator, the electrode and the sheath are adapted for use in combination with each other.

2. The system of claim 1, wherein the generator comprises at least one predefined parameter configured to indicate a position of the electrode, preferably of the distal end of the electrode when implanted in the septum of the heart.

3. The system of claim 1-2, wherein the generator comprises stimulation and/or diagnostic functions that are configured to be dependent upon a location of the distal end of the electrode.

4. The system of claim 1-3, wherein the fixing portion comprises a helical structure having a length of 0.1 to 5 mm, preferably 0.5 to 4 mm, preferably 1 to 3 mm, most preferably 1.5 to 1.8 mm, the helical structure configured to allow the distal end of the electrode to be screwed in the septum,
wherein the helical structure is preferably a piece separate from the electrode such that the helical structure can be at least partially removably attached to the electrode.

5. The system of claim 1-4, wherein the fixing portion comprises a first pole and the electrode comprises a second pole in proximity of the distal end, preferably between 1 to 10 mm, preferably 2 to 9 mm, preferably 3 to 8 mm, preferably 4 to 7 mm, preferably 5 to 6 mm spaced apart from the distal end, the first and the second pole configured to provide stimulation to the patient, wherein the electrode is preferably a bipolar electrode,
wherein the second pole has preferably the shape of a ring, preferably having a length along the longitudinal direction of the electrode of at least 0.5 mm, preferably at least 1 mm, preferably at least 1.5 mm, preferably at least 2 mm and/or of at most 5 mm, preferably at most 4 mm, preferably at most 3 mm, preferably at most 2 mm.

6. The system of claim 1-5, wherein the sheath has an inner surface facing the opening, wherein the inner surface is configured so as to facilitate guiding the electrode.

7. The system of claim 1-6, wherein the stimulation is configured to provide left bundle branch area pacing, wherein the system is preferably a conduction system pacing system.

8. The system of claim 1-7, wherein the generator is one or more of an implantable pulse generator, IPG, an implantable cardioverter defibrillators, ICD, a generator for cardiac resynchronization therapy with a defibrillator, CRT-D, a generator for cardiac resynchronization therapy with a pacemaker, CRT-P.

9. The system of claim 1-8, wherein the generator comprises a recess (11) for being engaged with the proximal end of the electrode, the recess comprising a first (12) and a second (13) pole for providing the stimulation to the electrode when the electrode is engaged with the recess,
wherein the generator preferably comprises a second recess (15) for being engaged with a further electrode, the second recess comprising a terminal, preferably an IS-1 terminal, wherein the further electrode is preferably an electrode configured to be implanted to provide stimulation to a left bundle branch area of the heart of the patient or
not to provide stimulation to a left bundle branch area of the heart of the patient.

10. The system of claim 1-9, wherein the generator comprises a programming interface comprising a switch configured to indicate whether or not the electrode is implanted in the left bundle branch area of the heart of the patient,
wherein the switch is configured to be activated when the electrode is implanted in the left bundle branch area of the heart of the patient.

11. The system of claim 10, wherein, when the switch is activated, the information that the electrode is implanted in the left bundle branch area of the heart of the patient is configured to be indicated to a human, such as the patient,
wherein preferably a brady parameter screen and/or a follow-up screen is/are configured to display this information,
wherein this information is preferably configured to be made available on programmer printouts,
wherein this information is preferably configured to be uploaded to remote monitoring system, HMSC.

12. The system of claim 10 or 11, wherein, when the switch is activated, a human such as the patient is allowed to enter left bundle branch, LBB, myocardial-only and/or Bundle branch block, BBB, correction capture threshold(s) separately or in combination for a substantially manual LV-threshold-test.

13. The system of claim 10-12, wherein providing stimulation to the patient is dependent on the information.

14. The system of claim 1-13, wherein the opening of the sheath comprises a stylet and/or wherein the sheath comprises a stylet, wherein the sheath is preferably a catheter.

15. The system of claim 1-14, wherein the sheath comprises a first deformation in a first plane and a second deformation in a second plane, the second plane being different from the first plane,
wherein the sheath is configured to be cut so as to facilitate removal of the sheath preferably after implanting the electrode, wherein preferably a tool can be used in order to cut the sheath.
